(19) Europäisches Patentamt
European Patent Office
Office européen des brevets

(11) Publication number: 0 247 526 A2

# (12) EUROPEAN PATENT APPLICATION

(21) Application number: 87107418.3

(51) Int. Cl.[4]: C25B 3/02 , C07C 45/58

(22) Date of filing: 21.05.87

(30) Priority: 22.05.86 IT 2052686

(43) Date of publication of application:
02.12.87 Bulletin 87/49

(84) Designated Contracting States:
AT BE CH DE ES FR GB LI NL

(71) Applicant: LARK S.p.A.
Via E. Romagnoli, 6
I-20146 Milan(IT)

(72) Inventor: Foa', Marco, Dr.
19, Via del Sabbione
I-28100 Novara(IT)
Inventor: Gatti, Norberto, Dr.
7, Via Gramsci
I-28066 Galliate Novara(IT)
Inventor: Forlini, Fabrizio, Dr.
67, Via Acerbi
I-27100 Pavia(IT)

(74) Representative: Weinhold, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

## (54) Process for 3,4-dimethoxyphenyl-acetone preparation.

(57) A process for preparing 3,4-dimethoxyphenylacetone is disclosed, which consists in electrolytically epoxidating isoeugenol-methylether in a mixture consisting of water and of a dipolar aprotic solvent, in the presence of $Br^-$ ions, and in isomerizing the so-obtained epoxide, in an inert organic solvent, in the presence of catalytic amounts of lithium salts, by means of a heating at temperatures comprised within the range of from 50°C up to the solvent refluxing temperatures.

EP 0 247 526 A2

## PROCESS FOR 3,4-DIMETHOXYPHENYLACETONE PREPARATION

The present invention relates to a process for the preparation of 3,4-dimethoxyphenylacetone by means of the electrochemical epoxidation of isoeugenol-methylether and subsequent catalytic isomerization of the so-obtained epoxide.

3,4-dimethoxyphenyl acetone is a useful intermediate for the synthesis of METHYLDOPA®, α-methyl-β-(3,4-dihydroxyphenyl)-alanine, an important antihypertensive agent (U.S.Pat. 2,868,818).

### STATE OF THE PRIOR ART

The preparation of 3,4-dimethoxyphenylacetone by means of the oxidation of isoeugenol-methylether by organic peracids (performic acid or peracetic acid), to yield an intermediate diol which is subsequently converted into a ketone by acidic hydrolysis is known (C.A. 82, 72640, 1975; and C.A. 69, 106243, 1968).

Such methods show however the drawback of requiring the use or organic peracids, the dangerousness of which is known.

Furthermore, the preparation is known of 3,4 -dimethoxyphenylacetone by starting from veratraldehyde via the Darzens reaction (C.A. 101, 152292, 1984), or by starting from 3,4-dimethoxyphenylacetic acid by condensation with acetic anhydride and ketene (C.A. 102, 24290, 1985).

Such methods, however, do not result economically valid, and they are difficultly accomplishable on an industrial basis, mainly due to the high cost and the not easy availability of the starting products.

Purpose of the present invention is preparing 3,4-dimethoxyphenylacetone by means of a simple, cheap, highly selective and high-yield process, which can be easily accomplished on an industrial scale by using non-dangerous reactants and low-cost, easily available starting products.

It has been found now that the above purpose, and still other purposes, are achieved by means of a process which comprises the electrochemical epoxidation of isoeugenol-methylether and the subsequent catalytic isomerization of the obtained epoxide into 3,4-dimethoxyphenylacetone.

### DESCRIPTION OF THE INVENTION

Therefore, the object of the present invention is a process for preparing 3,4-dimethoxyphenylacetone, characterized in that:

(a) isoeugenol-methylether, having the formula:

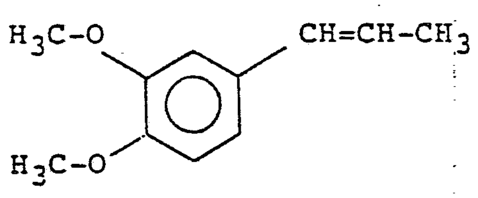

(I)

is submitted to an electrolysis in a not-partitioned electrochemical cell, in a medium comprising a dipolar aprotic solvent and an aqueous solution containing an alkali metal bromide or an alkali-earth metal bromide or a quaternary ammonium bromide, in such an amount as to have at least 0.6 mol of $Br^-$ ions per water litre, with graphite anodes or anodes constituted by titanium, coated with oxides of precious metals of the VIII group or with mixed oxides thereof with valve metals selected from Ti, Nb, Ta and Zr,

and that:

(b) the epoxide having the formula:

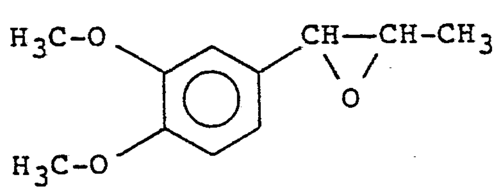

(II)

isolated from the reaction mixture resulting from (a) step is submitted to an isomerization, in an inert organic solvent and in the presence of catalytic amounts of a lithium salt selected from lithiun iodide, bromide and perchlorate, by being heated at temperatures comprised within the range of from 50°C to the solvent refluxing temperature.

The main reactions, concerning the process according to the present invention, can be schematically shown as follows:

(a)

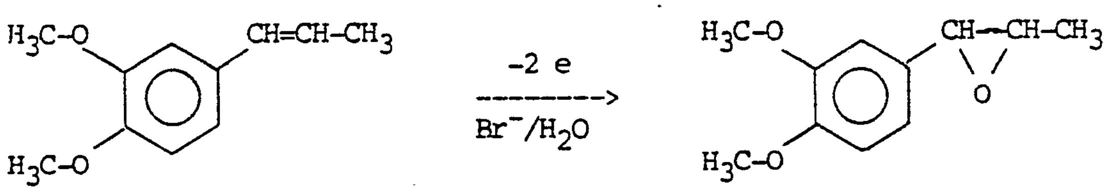


(I) (II)

(b)

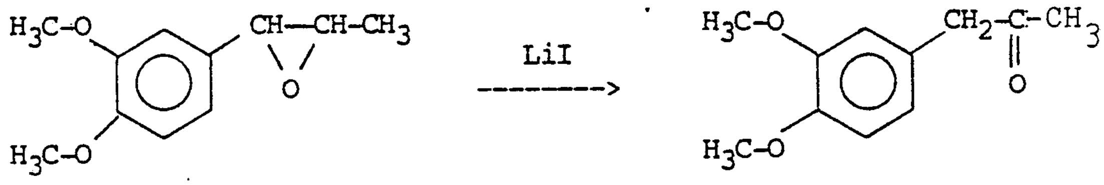


(II) (III)

The electrolysis reaction (a), leading to the formation of the epoxide, can be carried out both batchwise and continuously, at temperatures comprised within the range of from 0°C to 50°C, preferably of from l0°C to 30°C, with current intensities higher than l00 A/m² being used, and with the reaction mixture being kept stirred, by a stirring of mechanical type, or obtained by exploiting the turbolences as generated by the gases formed during the electrochemical reaction.

As the dipolar aprotic solvents, e.g., acetonitrile, dimethylformamide, dimethylsulphoxide, sulpholane, N-methylpyrrolidone and dimethylacetamide, preferably acetonitrile and dimethylformamide, can be used.

Generally, a volume ratio of the dipolar aprotic solvent to water comprised within the range of from l:l to l0:l and a concentration of isoeugenol-methylether (I) in the mixture constituted by the solvent and water higher than l0 g/l is used.

In order to be able to obtain high selectivities and high yields of epoxide (II), it was found in particular that using is necessary, in the electrolysis reaction (a), both a high concentration of $Br^-$ ions, higher than 0.6 mol/water litre, up to the concentration corresponding to the maximum solubility in $H_2O$ of the used bromide, and anodes constituted by graphite or titanium coated with oxides of precious metals of the VIII Group of the Periodic System, e.g., with Ru oxides or with mixed oxides of the same metals with such valve metals as Ti, Zr, Nb and Ta. In fact, the use of low concentrations of $Br^-$ ions and of common Pt anods leads to the formation of substantial amounts of byproducts, mainly constituted by dimerization products. The high concentration of bromides in the reaction mixture, besides favouring the yield of epoxide, renders easier the end separation of the epoxide from the organic reaction phase and allows furthermore high current efficiencies to be achieved.

The reaction (b) of isomerization of epoxide (II) to ketone (III), in the presence of catalytic amounts of a lithium salt, takes place with high yields and in a regioselective way. Generally, amounts of lithium salt comprised within the range of from 0.05 to 0.4 mol per mol of epoxide (II), and concentrations of epoxide in the organic solvent comprised within the range of from 5 to 50 g/l00 ml of solvent are used. As the inert organic solvents, e.g., acetonitrile and ($C_1$-$C_4$)-alkyl acetates, preferably ethyl acetate, can be used. The duration of the isomerization reaction can range from 2 up to l0 hours, according to the adopted experimental parameters.

According to a practical operating way, to a not-partitioned electrochemical cell the aqueous solution of alkali-metal bromide is charged, and to it the organic solution of olefin (I) is added; then, with the temperature being kept at a prefixed value comprised within the range of from I0°C to 30°C, a current amount comprised within the range of from 2 to 2.6 Faradays per olefin mole is passed, until the starting olefin has disappeared.

After the reaction has been completed, the epoxide (II) is separated from the aqueous phase containing the alkali-metal bromides.

The so-obtained epoxide is dissolved in the organic solvent selected for the subsequent isomerization step, e.g., in an alkyl acetate, to it a catalytic amount of lithium salt is added and the reaction mass is heated at the reflux temperature for the necessary time for isomerization to be completed.

The lithium salt is then separated from the organic phase by means of aqueous washes, or by the addition of a suitable non-solvent and subsequent filtration.

The ketone (III) is finally obtained by evaporating its organic solution to dryness, and shows, at a chromatographic analysis, a purity of ≥90%.

Then, if necessary, a further purification thereof may be performed by means of the common techniques, e.g., of distillation, liquid chromatography.

Some non-limitative examples are now supplied to the purpose of illustrating the invention.

## EXAMPLE I

6.27 g of NaBr is dissolved in 25 ml of $H_2O$ and I25 ml of $CH_3CN$, the mixture is then strongly stirred by means of magnetic stirring, and to it 3.76 g of isoeugenol-methylether (I) is then added.

The obtained mixture is then electrolysed in a 250-ml not-partitioned electrochemical cell, with a constant current of 850 mA, with two graphite anodes with a total surface of about I7 $cm^2$, and a central stainless-steel cathode having a surface of about 25 $cm^2$ being used, with a distance between electrodes of about I cm. 5,200 Coulombs are passed, with the reaction mixture being kept at a temperature of 20°C.

From the reaction mixture, discharged from the electrochemical cell, two phases, i.e., the aqueous phase, containing $Br^-$ ions, and the organic phase, containing acetonitrile and the reaction product, are separated.

From the organic phase acetonitrile is evaporated off under reduced pressure, and to the resulting reaction product 40 ml of ethyl acetate is added.

The gas-chromatographic analysis of the organic phase shows the presence of epoxide (II) with a >90% purity.

The reaction mixture in ethyl acetate is then transferred to a I00-ml reactor, purged under a nitrogen atmosphere, 340 mg of LiI is added, and the whole mass is then heated, with mechanical stirring, on an oil bath, up to ethyl acetate reflux temperature. The heating is continued for 5 hours, until the disappearance of the epoxide (II), as evidenced by the thin-layer chromatograpghy.

The reaction product is cooled to room temperature, is washed with I0 ml of $H_2O$ to the purpose of removing lithium iodide and is then dehydrated over $Na_2SO_4$.

3.57 g is obtained of dimethoxyphenylacetone (III), as determined by gas-chromatographic analysis with an inner standard of 4,4'-dimethoxybenzophenone. The yield of ketone (III) relatively to the olefin (I) used as the starting material is of 87.I%.

## EXAMPLE 2

Example I is repeated in exactly the same way, with the exception that in the isomerization step 250 mg of LiBr instead of 340 mg of LiI is used, and that the reaction time results to be of I0 hours, instead of 5 hours. In this way, a yield of ketone (III) of 86% relatively to the olefin (I) used as the starting material is obtained.

EXAMPLE 3

To a 250-ml not-partitioned electrochemical cell, I25 ml of $CH_3CN$, 25 ml of $H_2O$, 6.47 g of NaBr and 2.78 g of isoeugenol-methylether (I) is added. The mixture is electrolysed at a constant current of 350 mA, with a titanium anode coated with a mixed Ru-Ti oxide (50:50 by weight), with a total surface of about 7 $cm^2$, and a central stainless-steel cathode having a surface of about I5 $cm^2$ being used, with a distance between electrodes of about I cm. Through the cell 4,000 Coulombs are passed, with the reaction mixture being kept at the temperature of 20°C.

The reaction mixture is then processed according to such modalities as reported in Example I, until the solution of the reaction product in ethyl acetate is obtained; to such solution, 337 mg of LiI is added.

The mixture is then refluxed (at ethyl acetate refluxing temperature) for 5 hours, and the process is continued as described in Example I, until 2.795 g is obtained of ketone (III), with a yield of 92.2% relatively to the olefin (I) used as the starting material.

EXAMPLE 4

To a 250-ml not-partitioned electrochemical cell, I25 ml of $CH_3CN$, 25 ml of $H_2O$, 6.40 g of NaBr and 2.675 g of isoeugenol-methylether (I) is added.

The mixture, kept at 20°C, is electrolysed, with the same constant current density and the same set of electrodes as of Example I being used, through the cell 3,625 Coulombs, equalling 2.5 Faradays/mol, being passed. The reaction mixture is then transferred to a rotary evaporator, for $CH_3CN$ to be stripped under vacuum. The resulting reaction product is then extracted three times with 30 ml of ethyl acetate, and is then dried over $Na_2SO_4$.

The organic extract, concentrated to a volume of 25 ml, and with I60 mg of added LiI, is refluxed (at ethyl acetate refluxing temperature) for 6 hours.

The process is continued as described in Example I, and 2.54 g is obtained of ketone (III), with a yield of 86.5% relatively to the olefin (I) used as the starting material.

EXAMPLE 5

To a 250-ml not-partitioned electrochemical cell, I35 ml of $CH_3CN$, I5 ml of $H_2O$, 6.20 g of NaBr and 2.82 g of olefin (I) is added.

The mixture, kept at 20°C, is electrolysed by using the same electrodes as of Example I, but with a constant current density of I.7 A being used, until through the cell 4,000 Coulombs have been passed.

The reaction mixture is then processed as described in Example 4.

2.56 g is obtained of ketone (III), with a yield of 83.2%, as computed relatively to the olefin (I) used as the starting material.

EXAMPLES 6-9

To a 250-ml not-partitioned electrochemical cell, I00 ml of dimethylformamide, 50 ml of $H_2O$, 6.72 g of NaBr and 4.25 g of isoeugenol-methylether (I) is charged.

The mixture is then electrolysed under the same conditions, and by using the same set of electrodes as used in Example I, with a total of 5,670 Coulombs being passed.

At reaction end, the mixture is discharged, to it 250 ml is added of 20% aqueous NaCl solution, and it is then extracted four times with 50 ml of ethyl acetate. The extract is washed twice with 50 ml of 20% aqueous NaCl solution, and is then dried.

The organic extract is concentrated to a volume of I00 ml by the solvent being evaporated off.

On three aliquots, of 20 ml each, of said extract, the isomerization reactions are carried out at the ethyl acetate reflux temperature, by using the same lithium salts and reaction times as shown in Table I.

From the fourth aliquot of 20 ml of above said extract, ethyl acetate is evaporated off and replaced with the same amount of acetonitrile.

The isomerization of the reaction product is then carried out at acetonitrile refluxing temperature, with the lithium salt and the reaction time being used as shown in Table I.

In Table I also the conversions and the yields of ketone (III), as computed relatively to olefin (I) used as the starting material, are reported for each example.

TABLE 1

| Example | Solvent | Lithium Salt Type | Lithium Salt mg | Reaction Time, Hours | Conversion % | Yield % |
|---|---|---|---|---|---|---|
| 6 | Ethyl Acetate | LiI | 180 | 6 | 100 | 91.4 |
| 7 | Ethyl Acetate | LiBr | 140 | 10 | 100 | 87.7 |
| 8 | Ethyl Acetate | $LiClO_4$ | 160 | 10 | 60 | 45.8 |
| 9 | Acetronitrile | LiI | 185 | 6 | 100 | 77.1 |

## Claims

I. Process for preparing 3,4-dimethoxyphenylacetone, characterized in that:

(a) isoeugenol-methylether, having the formula:

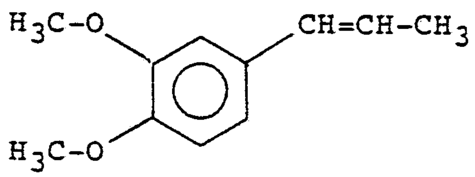

(I)

is submitted to an electrolysis in a not-partitioned electrochemical cell, in a medium comprising a dipolar aprotic solvent and an aqueous solution containing an alkali metal bromide or an alkali-earth metal bromide or a quaternary ammonium bromide, in such an amount as to have at least 0.6 mol of $Br^-$ ions per water litre, with graphite anodes or anodes constituted by titanium, coated with oxides of precious metals of the VIII group or with mixed oxides thereof with valve metals selected from Ti, Nb, Ta and Zr, and that:

(b) the epoxide having the formula:

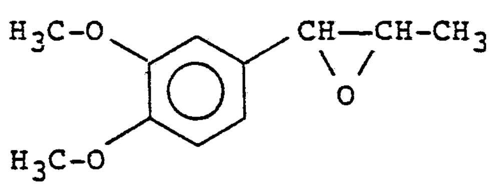

(II)

isolated from the reaction mixture resulting from (a) step is submitted to an isomerization, in an inert organic solvent and in the presence of catalytic amounts of a lithium salt selected from lithium iodide, bromide and perchlorate, by being heated at temperatures comprised within the range of from 50°C to the solvent refluxing temperature.

2. Process according to claim I, characterized in that the electrolysis is carried out at temperatures comprised within the range of from 0°C to 50°C, using a current density higher than I00 $A/m^2$.

3. Process according to one or both of the claims 1-2, characterized in that the ratio by volume of the dipolar aprotic solvent to water is comprised within the range of from 1:1 to I0:I and that the concentration of isoeugenol-methylether in the mixture constituted by the dipolar aprotic solvent and water is higher than 10 g/I.

4. Process according to claim 3, wherein the dipolar aprotic solvent is selected from acetonitrile and dimethylformamide.

5. Process according to one or more of the claims 1-4, wherein in (b) isomerization reaction an amount of lithium salt comprised within th range of from 0.05 to 0.4 mol/mol of epoxide (II), and a concentration of epoxide in the inert organic solvent comprised within the range of from 5 to 50 g/100 ml of solvent is used.

6. Process according to claim 5, characterized in that the inert organic solvent is selected from acetonitrile and ethyl acetate.